# EUROPEAN PATENT SPECIFICATION

(11) **EP 0 679 081 B1**
(45) Date of publication and mention of the grant of the patent: **06.02.2002**
(21) Application number: 95901556.1
(22) Date of filing: 08.11.1994
(51) Int. Cl.: A61K 31/195, A61K 31/375, A61K 31/70, A61K 31/405, A61K 31/19, A61K 31/525

(54) **PHARMACEUTICAL COMPOSITIONS FOR PREVENTION AND TREATMENT OF CANCEROUS DISEASE AND PROCESS FOR THEIR PREPARATION**
PHARMAZEUTISCHE ZUSAMMENSETZUNGEN ZUR PRÄVENTION UND BEHANDLUNG VON KREBSERKRANKUNGEN UND VERFAHREN FÜR IHRE HERSTELLUNG
COMPOSITIONS PHARMACEUTIQUES DESTINEES A LA PREVENTION ET AU TRAITEMENT DE MALADIES CANCEREUSES AINSI QUE LEUR PROCEDE DE PREPARATION

(30) Priority: 09.11.1993 HU 9303171
(43) Date of publication of application: 02.11.1995
(73) Proprietor: IMMUNAL KFT, 1055 Budapest (HU)
(72) Inventor: KULCSAR, Gyula, H-7632 Pécs (HU)
(74) Representative: Beszédes, Stephan G., Dr.
(86) International application number: HU9400049
(87) International publication number: WO9513061

(56) References cited:
- EP-A- 0 100 022
- EP-A- 0 178 602
- EP-A- 0 220 537
- EP-A- 0 347 714
- EP-A- 0 567 433
- WO-A-86/02555
- CH-A- 642 541
- DE-A- 2 021 969
- FR-A- 2 201 879
- FR-A- 2 244 468
- GB-A- 931 921
- PATENT ABSTRACTS OF JAPAN, Field C, Vol. 11, No. 365 (9C460), 1987; & JP,A,62 135 421 (MORIO KASAI); cited in the application.

## Description

This invention relates to the use of a composition for preparing medicaments for preventing and treating cancerous diseases and pharmaceutical compositions.

In cancer therapy the most frequently used methods are the surgical, the irradiation and the chemotherapeutical treatments *[The Pharmacological Basis of Therapeutics*, *Pergamon Press, Inc*., *New York, pp. 1202-1263 (1990), Harrison's Principles of Internat Medicine, 12th ed., International Edition, McGraw-Hill, Inc., New York, Vol 2, pp. 1587-1599 (1991); Scientific American Medicine, Scientific American, Inc., New York, Vol 2, 12(V), pp. 1-14 (1984)].* In irradiation therapy, beside the widely used ionizations, phototherapy and local hyperthermia combined with irradiation and chemotherapy are used in special cases, e.g. at carcinoma of skin. Categorized by their effects, origins and structures, among the tools of chemotherapy alkylating agents, plant alkaloids, antibiotics, antimetabolites, other remedies (e.g. asparaginase) and the often used different hormones can be found as well. The newly applied strategies in chemotherapy are the following: combination chemotherapy; long term, small dose venous or arterial infusion of chemotherapeutical drugs for reducing toxicity; high dose chemotherapy to overcome drug resistance and the same type of therapy combined with autologous transplantation of bone-marrow; chemotherapeutical drugs combined with biological response modifiers; use of adjuvant and neoadjuvant chemotherapy to a greater extent. The most frequently used biological response modifiers are: interferons, tumour necrosis factor, lymphokines, e.g. interleukin-2, and monoclonal antibodies. Different dietary methods, slightly elucidated serum preparations and the Simonton method using psychogenic effects belong to the currently applied methods in treatment of tumorous diseases, the effectiveness of which is not proved yet.

The most characteristic disadvantages of the currently applied methods detailed in the above references are: toxicity, significant adverse effects, low tumour specificity development of resistance and limited scope of effectiveness. Most cytotoxic drugs used in cancer therapy do not discriminate between neoplastic and normal proliferating cells; therefore, to avoid any irreversible damage to vital host tissues (e.g. bonemarrow, intestine) drugs must be administered in dosages which usually prove to be insufficient to eradicate all of the present neoplastic cells *[Pharmac. Ther., 49,* 43-54 *(1991)]*. Irradiation therapy can cause radiation injury and at the same time it is not effective at hypoxial cells and certain type of tumours. Drugs used in chemotherapy have also various toxic side effects. They can damage the central nervous system, haematopoietic organs, mucous membranes of stomach and bowels and all proliferating cells. In addition they can harm liver, kidney, lung and cardiac muscle as well. Almost all effective antitumour agents are immunosuppressive *[Proc*. *Roy*. *Soc*. *Med*., *63, 1063-1066 (1970)]*. Many of them have teratogenic or carcinogenic effects, occasionally they can cause infertility or increase the frequency of the secondary rise of tumours. It is difficult or impossible to influence 60-70% of the tumours by chemotherapy and during treatment a resistance or cross-resistance may develop, too.

Unfortunately, treatments using biological response modifyers utilizing the own defence mechanism of the organism have similar disadvantages since they have been found to be effective only in a few types of tumours and besides they have also toxic side effects *[Harrison's Principles of internal Medicine*, *12th ed*., *International Edition*, *McGraw-Hill*, *Inc*., *New York, Vol 2, pp*. *1587-1599 (1991)]*. Interferons have also *many and* severe adverse effects, among other things e.g. cardiotoxicity *[Chest*, *99, 557-561 (1991)]*. Similarly, the hopes attached to monoclonal antibodies also failed to materialize *[Eur*. *J. Cancer, 27, 936-939 (1991)]*. Although since 1980 more than four hundred registered clinical trials of immunotherapy have been performed, no one has been introduced yet as a treatment for any type of cancer *[J*. *R.* *Soc*. *Med*., *84, 321 (1991)]*.

The PCT publication No. WO 86/02555 relates to a drug used in treatment of cancerous diseases comprising L-cysteine, L-methionine, L-histidine, L-phenylalanine, L-lysine, L-tryptophan, L-valine, L-leucine and L-threonine as well as L-malic acid and L-ascorbic acid. L-malic acid and L-ascorbic acid as activators buffer and stabilize the amino acids in their acid form and thus they cause a modification of the blood pH value in the acidity range under 6.8. In this publication there are no data confirming growth stop or even regression of malignant tumours.

From EP-A-0347 714 there have been known dialysing and rinsing solutions for the intraperitoneal administration comprising the amino acids L-histidine, L-isoleucine, L-leucine, L-methionine, L-valine, L-lysine hydrochloride, L-phenylalanine, L-threonine, L-tyrosine, L-taurine and L-tryptophan, vitamins, such as biotin or vitamin C, and malate. However, no mention has been made of an activity of these for preventing or treating cancerous diseases.

In DE 2 021 969 there has been described a process for preparing preparations free from proteins containing amino acids, ascorbic acid and orotic acid. However, no activity for preventing or treating cancerous diseases has been mentioned either for these compositions.

Furthermore from EP-A-0567 433 there have been known orally ingestible nutrition compositions having improved palatibility and containing the amino acids arginine or valine, malate and vitamin C. However, no activity for preventing or treating cancerous diseases has been disclosed for these compositions either.

The published Japanese patent application No. 62-135 421 relates to an amino acid transfusion solution containing specific amino acids, with the exception of L-isoleucine, capable of exhibiting inhibitory action on tumour multiplication. The possible but unproved effect of this preparation might base on an amino acid, e.g. isoleucine, restriction.

The aim of the present invention is to provide the use of a composition comprising natural substances for preparing medicaments for preventing and treating cancerous diseases and new pharmaceutical compositions to eliminate the disadvantages, e.g. toxicity, low specificity and limited scope of effectiveness, of the compositions used according to the Prior Art and known compositions.

The invention is based on the recognition of a passive defence system [hereinafter called Passive Antitumour Defence System (PADS)] against tumour cells in different organisms. This system is able to destroy the arising and already existing tumour cells. The substances participating in PADS are endogenous and exogenous natural substances occurring in the circulatory system, namely amino acids, vitamins, nucleic bases, carbohydrates and cell-metabolic products. It has been recognized that using together at least three from these substances - which are components of the circulatory system and thus can reach and enter all cells - they synergistically increase the effect of each other and thus are able to destroy tumour cells.

The invention is further based on the recognition that due to the synergism there will be a significant qualitative difference between normal and tumour cells in their behavior towards the participants of PADS whereby the two kinds of cells become distinguishable. While the classical immune system can recognize and selectively destroy tumour cells because of their external deviations from normal cells, the newly recognised passive antitumour defence mechanism can do the same in consequence of internal deviations.

The invention is further based on the recognition that, due to the increase of the concentration of its components in the circulatory system to such a degree as to be oversupplied for tumour cells, the use of the above-mentioned composition causes a fatal metabolic stress selectively in cancer cells.

The invention is finally based on the recognition that depending on the applied dosage and the way of application, a preventing or anti-tumour effect can be achieved in different tumour cells. In case of various types of tumours that differ from normal cells in different degrees, the qualitative and quantitative composition of the most effective mixture can be determined using the synergism as a criteria.

Based on the above, the invention relates to the use of a composition for preparing a medicament for preventing and treating cancerous diseases, the said composition comprising at least three active compounds occurring in the circulatory system: at least one amino acid, at least one vitamin and at least one member selected from the group consisting of adenine, 2-deoxy--D-ribose, D-mannose, malic acid and/or their pharmaceutically acceptable salts, with the proviso that if the composition contains beside amino acid(s) only malic acid and a vitamin, the vitamin may be only other than ascorbic acid.

The composition used may comprise also carriers, diluents and/or other auxiliary agents commonly used in pharmacy.

In the use according to the invention advantageously L-methionine, L-tryptophan, L-tyrosine, L-phenylalanine, L-arginine, L-histidine, N-benzoylglycine and/or a salt thereof is employed as amino acid and d-biotin, pyridoxine, riboflavin, riboflavin-5'-phosphate, L-ascorbic acid, orotic acid and/or a salt thereof is employed as vitamin.

According to a preferred embodiment of the invention 0.9-25% by mass of L-methionine, 0.8-19% by mass of L-tryptophan, 1.1-48% by mass of L-arginine, 0.9-46% by mass of d-biotin, 1.2-16% by mass of pyridoxine, 0.03-42% by mass of riboflavin-5'-phosphate, 0.05-18% by mass of D-glucosamine, 0.5-60% by mass of 2-deoxy-D-ribose, 0.7-68% by mass of malic acid, 0.6-40% by mass of D-mannose and/or their pharmaceutically acceptable salts are employed.

According to another preferred embodiment of the invention 0.005-34% by mass of L-methionine, 0.002-25% by mass of L-tryptophan, 0.02-23% by mass of L-tyrosine, 0.04-30% by mass of L-phenylalanine, 0.04-50% by mass of L-arginine, 0.03-34% by mass of L-histidine, 0.05-22% by mass of N-benzoylglycine, 0.01-60% by mass of d-biotin, 0.01-20% by mass of pyridoxine, 0.0004-45% by mass of riboflavin, 0.0005-45% by mass of riboflavin-5'-phosphate, 0.003-70% by mass of L-ascorbic acid, 0.004-15% by mass of lipoic acid, 0.01-17% by mass of orotic acid, 0.001-10% by mass of adenine, 0.01-63% by mass of 2-deoxy-D-ribose, 0.08-42% by mass of D-mannose, 0.05-20% by mass of D-glucosamine, 0.01-80% by mass of malic acid, 0.02-60% by mass of oxalacetic acid, 0.001-10% by mass of adenosine triphosphate and/or their pharmaceutically acceptable salts are employed.

According to a further preferred embodiment of the invention 30-44% by mass of L-arginine, 27-35% by mass of riboflavin-5'-phosphate, 38-62% by mass of malic acid and/or their pharmaceutically acceptable salts are employed.

The usual drug formulations prepared by the use according to the invention can be oral compositions in solid or liquid form, e.g. tablets, capsules, powders, dragées, pills, granules, syrups, solutions, emulsions or suspensions; or topically used ointments, cremes for external treatment; or rectal compositions such as suppositories; as well as parenteral compositions, e.g. injectable solutions or infusions.

According to an advantageous embodiment of the invention its use is for preparing medicaments in the form of tablets or capsules for oral administration.

According to a further advantageous embodiment of the invention its use is for preparing medicaments in the form of an infusion solution for parenteral administration. Preferably this use is for preparing 0.5 to 3 l of an infusion solution comprising 10 to 200 g/l of the active composition. Particularly it is an infusion solution comprising 50 to 190 g/l of the active composition, above all an infusion solution comprising 150 to 180 g/l of the active composition.

The formulations prepared by the use according to the invention are suitably prepared in such a way that, after having mixed the active compounds with non-toxic inert, solid or liquid carriers, diluents, binding agents and/or other additives commonly used in the pharmaceutical technique for enteral or parenteral administration, they are transformed to one of the usual drug formulations.

A subject-matter of the invention are also pharmaceutical compositions comprising 30-44% by mass of L-arginine, 27-35% by mass of riboflavin-5'-phosphate, 38-62% by mass of malic acid and/or their pharmaceutically acceptable salts.

The above preferences of forms likewise apply for these compositions.

The preferable daily dose of the compositions used according to the invention depends on several factors such as the nature of the illness to be treated, the state of the patient, the way of treatment etc. The preferable daily dose for curative purposes amounts to 30-3000 mg/kg body weight, more preferably 400-2500 mg/kg, most preferably 1000-2000 mg/kg. Accordingly, it is suitable to administer daily 0.5-3 l of infusion solutions containing preferably 10-200 g/l, more preferably 25-120 g/l, most preferably 40-75 g/l of the active composition. The preferable daily dose for preventive purposes amounts to 5-100 mg/kg body weight, more preferably 15-80 mg/kg, most preferably 30-60 mg/kg. Accordingly, it is suitable to administer daily 1-8, more preferably 2-6, most preferably 3-4 tablets, capsules or dragées each containing 0.2-2 g, more preferably 0.5-1.5 g, most preferably 0.75-1 g of the active composition or 0.5-3 l of infusion solutions containing preferably 10-200 g/l, more preferably 50-190 g/l, most preferably 150-180 g/l of the active composition.

The medicaments prepared by the use according to the invention should be administered in a therapeutically effective amount to the patient to be treated.

The invention will be elucidated in more detail in the following Tables, Figures and Examples. The cells used in the following experiments were received from the "American Type Culture Collection" (Rockville, MD, USA).

Table 1 shows the tumour cell killing effects and synergistic co-operation of components of compositions of Examples 1-20 comprising four and five active components, resp., on Sp2/0-Ag14 myeloma cells (ATCC CRL 1581).

Table 2 shows, based on Examples 21-33, the increase of the effect of a composition comprising five active components on Sp2/0-Ag14 cells when further components are added.

Figure 1 shows the effects and synergistic co-operation of components of compositions comprising four and five active components, resp., according to Examples 1-20.

Figure 2 compares the in vitro effects of compositions comprising five active components according to Examples 98-102 and of compositions comprising twenty one active components according to Examples 103-107 on Sp2/0-Ag14 mouse myeloma cells in relation to a corresponding control mixture.

**Figure 3** shows the effect of compositions comprising seventeen active components according to Example 103 on Sp2/0-Ag14 mouse myeloma cells as a function of time in relation to untreated cells and to a suitable control mixture.

**Figure 4** shows the in vitro effects of compositions comprising seventeen active components according to Examples 103-107 on K-562 human erythroleukaemia cells (ATCC CCL 243) in relation to corresponding control mixtures.

**Figure 5** shows the in vitro effects of compositions comprising seventeen active components according to Examples 103-107 on HeLa human epitheloid carcinoma cervix cells (ATCC CCL 2) in relation to corresponding control mixtures.

**Figure 6** shows the in vitro effects of compositions comprising seventeen active components according to Examples 103-107 on HEp-2 human epidermoid carcinoma, larynx cells (ATCC CCL 23) in relation to corresponding control mixtures.

**Figure 7** shows the in vitro effects of compositions comprising seventeen active components according to Examples 103-107 on normal Vero African green monkey kidney cells (ATCC CCL 81) in relation to corresponding control mixtures.

**Figure 8** shows the in vivo effects of compositions comprising seventeen active components according to Example 108 on a tumour developed from Sp2/0-Ag14 mouse myeloma cells injected i.p. in BALB/c mice in relation to a corresponding control group.

**Figure 9** shows the in vivo effects of compositions comprising seventeen active components according to Example 108 on solid tumour developed under skin from HeLa human epitheloid carcinoma cervix cells injected s.c. in BALB/c (nu/nu) mice in relation to a corresponding control group.

For the experiments the following media were used: in case of Sp2/0-Ag14 and K-562 cells RPMI 1640 medium (Sigma Chemie GmbH, D-8024 Deisenhofen, Germany, product number: R 6504), in case of HEp-2, HeLa and Vero cells MEM medium (Sigma Chemie GmbH, product number: M 4655).

### Examples 1-33

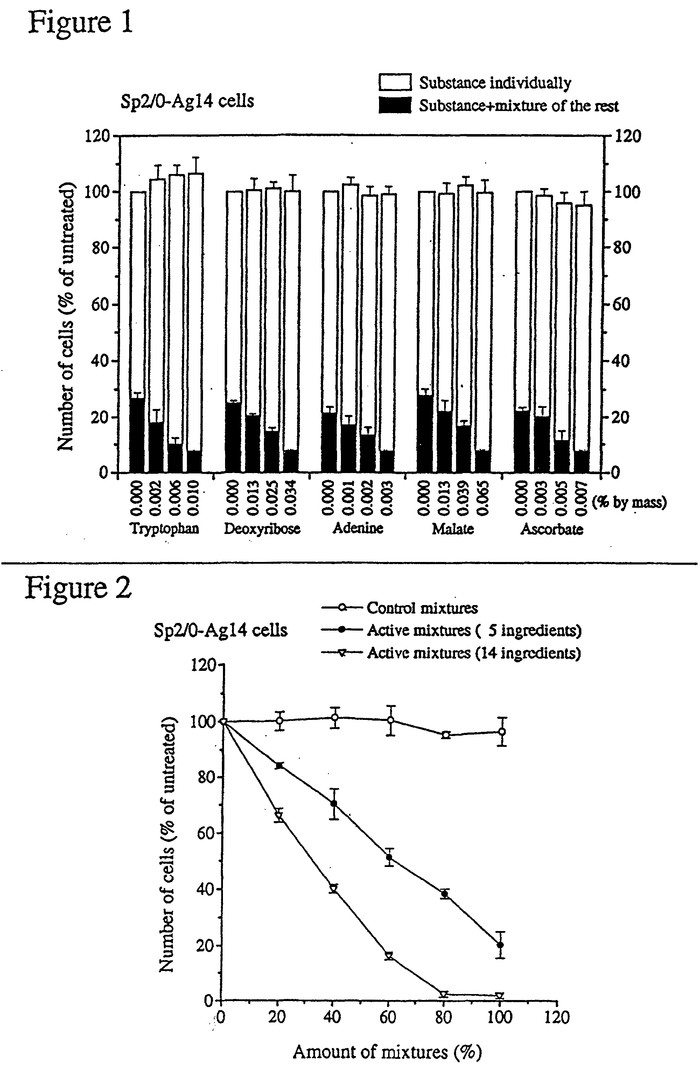

Into an apparatus equipped with a stirrer active agents in quantities given and shown in Tables 1 and 2 are weighed in, then to the obtained powder mixture the amounts of sodium hydrogen carbonate given also in Tables 1 and 2 necessary for the neutralization of the acidic-type components are added. While continuous stirring the appropriate medium is added to the mixture to supplement the mass of the composition to 100 %. The effects of solutions prepared in this way can be seen in Tables 1 and 2 and in Figure 1.

### Examples 34-63

One proceeds according to Examples 1-33 with the difference that for the neutralization of the acidic-type components suitable amounts of potassium hydrogen carbonate are used, instead of sodium hydrogen carbonate. The effects of solutions prepared in this way do not differ significantly in any case from the effects of the solutions of Examples 1-33 shown in Tables 1 and 2 and in Figure 1, respectively.

### Examples 64-93

One proceeds according to Examples 1-33 with the difference that for the neutralization of the acidic-type components suitable amounts of calcium carbonate are used, instead of sodium hydrogen carbonate. The effects of solutions prepared in this way do not differ significantly in any case from the effects of the solutions of Examples 1-33 shown in Tables 1 and 2 and in Figure 1, respectively.

### Examples 94-97

One proceeds according to Examples 23, 24, 27 and 28 with the difference that 0.053 % by mass of L-arginine hydrochloride, 0.052 % by mass of L-histidine hydrochloride, 0.009 % by mass of L-methionine hydrochloride and 0.025 % by mass of L-tyrosine hydrochloride, resp., are used, instead of L-arginine, L-histidine, L-methionine and L-tyrosine, resp., used in Examples 23, 24, 27 and 28, resp. The corresponding amount of sodium hydrogen carbonate was 0.054 % by mass in each case. The effects of solutions prepared in this way do not differ significantly from the effects of the solutions of Examples 23, 24, 27 and 28 shown in Table 2.

### Example 98

Into an apparatus equipped with a stirrer 0.01 % by mass of L-tryptophan, 0.034 % by mass of 2-deoxy-D-ribose, 0.003 % by mass of adenine, 0.065 % by mass of malic acid, 0.007 % by mass of L-ascorbic acid and 0.091 % by mass of sodium hydrogen carbonate are weighed in. Then while continuous stirring the appropriate medium is added to this mixture to supplement the mass of the composition to 100%. The effect of the solution prepared in this way is shown in Figure 2 at the 100 % composition.

### Example 99

One proceeds according to Example 98 with the difference that only 80 % of the quantities are weighed in from every component. Then the appropriate medium is added to this mixture to supplement the mass of the composition to 100%. The effect of the solution prepared in this way is shown in Figure 2 at the 80 % composition.

### Example 100

One proceeds according to Example 98 with the difference that only 60 % of the quantities are weighed in from every component. Then the appropriate medium is added to this mixture to supplement the mass of the composition to 100%. The effect of the solution prepared in this way is shown in Figure 2 at the 60 % composition.

### Example 101

One proceeds according to Example 98 with the difference that only 40 % of the quantities are weighed in from every component Then the appropriate medium is added to this mixture to supplement the mass of the composition to 100%. The effect of the solution prepared in this way is shown in Figure 2 at the 40 % composition.

### Example 102

One proceeds according to Example 98 with the difference that only 20 % of the quantities are weighed in from every component. Then the appropriate medium is added to this mixture to supplement the mass of the composition to 100%. The effect of the solution prepared in this way is shown in Figure 2 at the 20 % composition.

### Example 103

Into an apparatus equipped with a stirrer 0.011 % by mass of L-methionine, 0.01 % by mass of L-tryptophan, 0.036 % by mass of L-tyrosine, 0.041 % by mass of L-phenylalanine, 0.044 % by mass of L-arginine, 0.039 % by mass of L-histidine, 0.007 % by mass of L-ascorbic acid, 0.012 % by mass of d-biotin, 0.010 % by mass of pyridoxine, 0.0004 % by mass of riboflavin, 0.0006 % by mass of riboflavin-5'-phosphate, 0.003 % by mass of adenine, 0.017 % by mass of orotic acid, 0.089 N-benzoylglycine, 0.034 % by mass of 2-deoxy-D-ribose, 0.090 % by mass of D-mannose, 0.065 % by mass of malic acid and 0.087 % by mass of sodium hydrogen carbonate are weighed in. Then while continuous stirring the appropriate medium is added to this mixture to supplement the mass of the composition to 100%. The effect of the solution prepared in this way is shown in Figures 2, 3, 4, 5, 6 and 7 at the 100 % composition.

### Example 104

One proceeds according to Example 103 with the difference that only 80 % of the quantities are weighed in from every component Then the appropriate medium is added to this mixture to supplement the mass of the composition to 100%. The effect of the solution prepared in this way is shown in Figures 2, 3, 4, 5, 6 and 7 at the 80 % composition.

### Example 105

One proceeds according to Example 103 with the difference that only 60 % of the quantities are weighed in from every component Then the appropriate medium is added to this mixture to supplement the mass of the composition to 100%. The effect of the solution prepared in this way is shown in Figures 2, 3, 4, 5, 6 and 7 at the 60 % composition.

### Example 106

One proceeds according to Example 103 with the difference that only 40 % of the quantities are weighed in from every component Then the appropriate medium is added to this mixture to supplement the mass of the composition to 100%. The effect of the solution prepared in this way is shown in Figures 2, 3, 4, 5, 6 and 7 at the 40 % composition.

### Example 107

One proceeds according to Example 103 with the difference that only 20 % of the quantities are weighed in from every component. Then the appropriate medium is added to this mixture to supplement the mass of the composition to 100%. The effect of the solution prepared in this way is shown in Figures 2, 3, 4, 5, 6 and 7 at the 20 % composition.

### Example 108

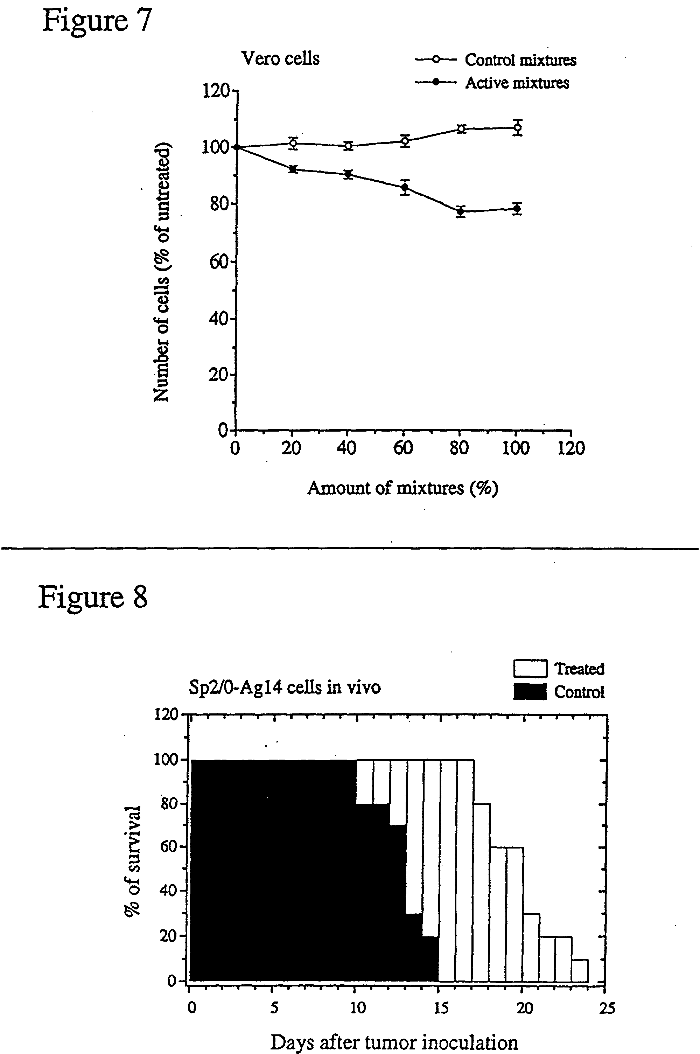

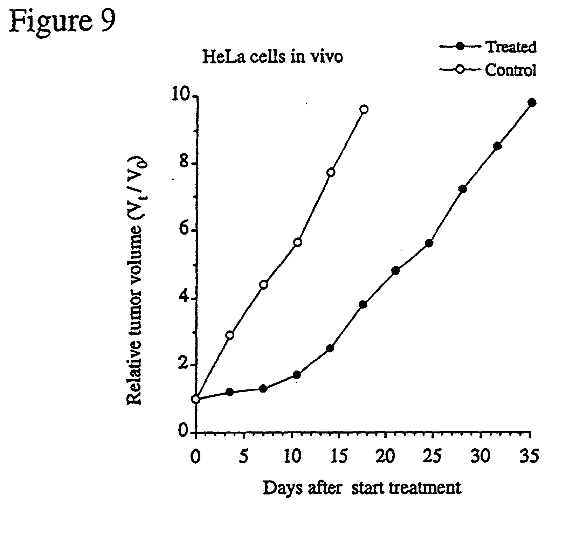

Into an apparatus equipped with a stirrer 1.47 % by mass of L-methionine, 1.01 % by mass of L-tryptophan, 0.036 % by mass of L-tyrosine, 1.63 % by mass of L-phenylalanine, 1.71 % by mass of L-arginine, 1.53 % by mass of L-histidine, 1.94 % by mass of L-ascorbic acid, 1.21 % by mass of d-biotin, 2.02 % by mass of pyridoxine, 0.038 % by mass of riboflavin, 0.05 % by mass of riboflavin-5'-phosphate, 0.068 % by mass of adenine, 0.09 % by mass of orotic acid, 0.18 % by mass of N-benzoylglycine, 1.32 % by mass of 2-deoxy-D-ribose, 1.8 % by mass of D-mannose, 1.32 % by mass of malic acid, 1.34 % by mass of sodium hydrogen carbonate and 0.04 % by mass of potassium hydrogen carbonate are weighed in. While continuous stirring 81.198 % by mass of a buffer containing 0.02 % by mass of KH₂PO₄ and 0.3 % by mass of Na₂HPO₄ is added to this mixture. The effect of the solution prepared in this way and used for the treatment of animals is shown in Figures 8 and 9.

### Example 109

Into a mixer 20 % by mass of L-tryptophan, 62 % by mass of L-ascorbic acid and 18 % by mass of D-mannose are weighed in. After thorough mixing the resulting powder mixture is used for preventive experiments.

### Example 110

One proceeds according to Example 109 with the difference that 32 % by mass of L-arginine, 28 % by mass of riboflavin-5'phosphate and 40 % by mass of malic acid are used.

### Example 111

One proceeds according to Example 109 with the difference that 23 % by mass of L-tryptophan, 0.2 % by mass of pyridoxine, 17.8 % by mass of N-benzoylglycine and 59 % by mass of malic acid are used.

### Example 112

One proceeds according to Example 109 with the difference that 19 % by mass of L-tyrosine, 61 % by mass of L-ascorbic acid, 0.2 % by mass of adenine and 19.8 % by mass of L-histidine are used.

### Example 113

One proceeds according to Example 109 with the difference that 31 % by mass of L-methionine, 53 % by mass of d-biotin, 0.2 % by mass of adenine and 15.8 % by mass of orotic are used.

### Example 114

One proceeds according to Example 109 with the difference that 27 % by mass of L-phenylalanine, 33 % by mass of riboflavin, 0.2 % by mass of orotic acid and 39.8 % by mass of D-mannose are used.

### Example 115

One proceeds according to Example 109 with the difference that 32 % by mass of L-histidine, 9 % by mass of d-biotin acid and 59 % by mass of 2-deoxy-D ribose are used.

### Example 116

One proceeds according to Example 109 with the difference that 12 % by mass of L-arginine, 11 % by mass of pyridoxine and 77 % by mass of malic acid are used.

### Example 117

Into a mixer 10 % by mass of L-methionine, 3 % by mass of L-tryptophan, 0.2 % by mass of L-tyrosine, 11 % by mass of L-phenylalanine, 23.8 % by mass of L-arginine, 10 % by mass of L-histidine, 14.9 % by mass of L-ascorbic acid, 0.1 % by mass of d-biotin, 0.2 % by mass of pyridoxine, 0.05 % by mass of riboflavin, 0.35 % by mass of riboflavin-5'-phosphate, 0.3 % by mass of adenine, 0.6 % by mass of orotic acid, 1.1 % by mass of N-benzoylglycine, 0.9 % by mass of 2-deoxy-D-ribose, 11.5 % by mass of D-mannose and 12 % by mass of malic acid are weighed in. After thorough mixing the resulting powder mixture is used for preventive experiments.

The effects of the compositions of Examples 1-33 and the synergistic co-operation of the active ingredients (compared to the effects of the components shown alone) were investigated on Sp2/0-Ag14 mouse myeloma cells.

In the experiments the newest methods approved by the scientific literature were used. In case of Sp2/0-Ag14 and K562 lines, the logarithmically growing cells were harvested from the medium and resuspended in a 96-well plate used for cell culture to a final concentration of 4x10⁴ Sp2/0-Ag14 cells and 2x10⁴ K562 cells, respectively, in 250 µl appropriate medium per well containing the tested materials in the indicated concentrations. In case of HeLa, HEp-2 and Vero cells, the cultured cells were harvested from 75 % confluent tissue culture flasks with 0.2 % trypsin and resuspended in the appropriate medium at a density of 10⁵ cells/ml. Aliquots (100 µl) were dispensed into 96-well micro plates and incubated for 24 hours. Then the medium was discarded and replaced with 250 µl fresh medium containing the tested compounds in the indicated concentrations. All kinds of cells were allowed to proliferate for 48 hours. The number of viable Sp2/0-Ag14 and K562 cells was then counted microscopically with the trypan blue dye exclusion method. The survival of HeLa, HEp-2 and Vero cells was measured by assessing the endogenous alkaline phosphatase activity of the cells. Results were evaluated using Student's t-test. Data after the mean values in the Tables and signs in the Figures mean: "standard error of mean" (SEM).

The first column of Table 1 shows the Example number, the second one shows the number of control experiments while in the further six columns quantities of components used in the experiments and expressed in % by mass are shown. The control mixtures were composed of the same amounts of chemically similar but pharmacologically ineffective substances (0.026 % by mass of L-serine, 0.033 % by mass of L-asparagine, 0.029 % by mass of L-valine, 0.018 % by mass of L-alanine, 0.006 % by mass of glycine and 0.006 % by mass of L-proline as amino acids; 0.017 % by mass of thiamine hydrochloride, 0.006 % by mass of niacin, 0.019 % by mass of folic acid sodium salt, 0.001 % by mass of D-pantothenic acid hemicalcium salt as vitamins; 0.003 % by mass of hypoxanthine, 0.012 % by mass of uracil, 0.059 % by mass of trimethylglicine, 0.038 % by mass of D(-)ribose, 0.090 % by mass of glucose, 0.081 % by mass of succinic acid disodium salt,) as the active composition. The two last columns show the effects of compositions comprising four and five active ingredients, resp., and the effects of the components per se (control experiments), namelythe number of cells after 48 hr incubation and the amount of cells expressed in percentage of the amount of untreated cells, respectively (the amount of untreated cells is 100 %). In case of L-tryptophan for example the 2-4th control experiments show the effects of 0.002; 0.006; and 0.01 % by mass, resp., of L-tryptophan alone on the number of cells. In case of Examples 2-4 the effect of L-tryptophan used in the same amounts but applied together with the other four active ingredients is shown.

It can be seen from data of the two last columns of Table 1 that on using the above-mentioned substances alone neither of them showed tumour-cell-killing effect: in fact, using the given amounts of L-tryptophan the proliferation of cells even increased slightly. At the same time it can also be seen from the data of the columns in question that every substance increased the effect of the other four active ingredients proportionally to its amount in a synergistic manner. For example 0.01 % by mass of L-tryptophan increased the 73.1 % effect of the four other active ingredients to 92.3 %. The same amount (0.01 % by mass) of L-tryptophan itself, without the other four components, even increased slightly the proliferation of cells. This proves unambiguously a synergistic effect.

The difference between the effects of compositions comprising four active ingredients (Examples 1, 5, 9, 13 and 17) and of compositions comprising the fifth active ingredient (Examples 4, 8, 12, 16 and 20) in the maximum amounts used here (e.g. in case of L-tryptophan 0.01 % by mass) was significant in all cases (p<0.01), thus all substances significantly increased the effects of the other four components.

**Table 1**

| Effects of compositions of the invention and the individual active ingredients on Sp2/0-Ag14 mouse myeloma cells | | | | | | | | | |
|---|---|---|---|---|---|---|---|---|---|
| **EXAMPLE** | **CONTROLS** | **AMOUNTS OF COMPONENTS IN % BY MASS** | | | | | | **EFFECT** | |
| | | L-TRYPTOPHAN | 2-DEOXYRIBOSE | ADENINE | MALIC ACID | ASCORBIC ACID | Na-BICARBONATE | CHANGE OF THE NUMBER OF CELLS (x 10⁴) | IN% OF THE UNTREATED CELLS |
| 1 | | ― | 0.034 | 0.003 | 0.065 | 0.007 | 0.091 | 15.9±1.2 | 26.9±2.0 |
| | 1 | ― | ― | ― | ― | ― | ― | 59.1 ±3.2 | 100.0 |
| 2 | | 0.002 | 0.034 | 0.003 | 0.065 | 0.007 | 0.091 | 10.7±2.9 | 18.1±4.8 |
| | 2 | 0.002 | ― | ― | ― | ― | ― | 61.7±3.0 | 104.4±5.0 |
| 3 | | 0.006 | 0.034 | 0.003 | 0.065 | 0.007 | 0.091 | 6.0±1.4 | 10.1 ±2.4 |
| | 3 | 0.006 | ― | ― | ― | ― | ― | 62.6±2.0 | 106.0±3.4 |
| 4 | | 0.010 | 0.034 | 0.003 | 0.065 | 0.007 | 0.091 | 4.5±0.3 | 7.7±0.6 |
| | 4 | 0.010 | ― | ― ― | ― | ― | ― | 63.0±3.4 | 106.6±5.8 |
| 5 | | 0.010 | ― | 0.003 | 0.065 | 0.007 | 0.091 | 14.8 ±0.4 | 25.1 ±0.7 |
| | 5 | ― | ― | ― | ― | ― | ― | 59.1±3.2 | 100.0 |
| 6 | | 0.010 | 0.013 | 0.003 | 0.065 | 0.007 | 0.091 | 12.2±0.3 | 20.6±0.6 |
| | 6 | ― | 0.013 | ― | ― | ― | ― | 59.3±2.3 | ±4.1 |
| 7 | | 0.010 | 0.025 | 0.003 | 0.065 | 0.007 | 0.091 | 8.7±0.8 | 14.8±1.3 |
| | 7 | ― | 0.025 | ― | ― | ― | ― | 59.8±1.2 | 101.1±2.2 |
| 8 | | 0.010 | 0.034 | 0.003 | 0.065 | 0.007 | 0.091 | 4.5±0.3 | 7.7±0.6 |
| | 8 | ― | 0.034 | ― | ― | ― | ― | 59.0 ±3.2 | 99.9 ±5.9 |
| 9 | | 0.010 | 0.034 | ― | 0.065 | 0.007 | 0.091 | 12.6±1.3 | 21.4±2.3 |
| | 9 | ― | ― | ― | ― | ― | ― | 59.1±3.2 | 100.0 |
| 10 | | 0.010 | 0.034 | 0.001 | 0.065 | 0.007 | 0.091 | 10.1±1.8 | 17.1±3.2 |
| | 10 | ― | ― | 0.001 | ― | ― | ― | 60.5±1.4 | 102.4±2.5 |
| 11 | | 0.010 | 0.034 | 0.002 | 0.065 | 0.007 | 0.091 | 7.9 ± 1.7 | 13.4±2.9 |
| | 11 | ― | ― | 0.002 | ― | ― | ― | 58.2 ±1.8 | 98.5 ±3.2 |
| 12 | | 0.010 | 0.034 | 0.003 | 0.065 | 0.007 | 0.091 | 4.5 ± 0.3 | 7.7 ± 0.6 |
| | 12 | ― | ― | 0.003 | ― | ― | ― | 58.6 ± 1.5 | 99.1 ± 2.8 |
| 13 | | 0.010 | 0.034 | 0.003 | ― | 0.007 | 0.007 | 16.6 ± 1.2 | 28.1 ± 2.1 |
| | 13 | ― | ― | ― | ― | ― | ― | 59.1 ±3.2 | 100.0 |
| 14 | | 0.010 | 0.034 | 0.003 | 0.013 | 0.007 | 0.024 | 13.1 ± 2.2 | 22.2 ± 4.0 |
| | 14 | ― | ― | ― | 0.013 | ― | ― | 58.7 ± 2.1 | 99.3±3.7 |
| 15 | | 0.010 | 0.034 | 0.003 | 0.039 | 0.007 | 0.057 | 9.9 ± 1.1 | 16.7 ± 2.0 |
| | 15 | ― | ― | ― | 0.039 | ― | ― | 60.4 ± 1.7 | 102.2 ± 3.0 |
| 16 | | 0.010 | 0.034 | 0.003 | 0.065 | 0.007 | 0.091 | 4.5±0.3 | 7.7±0.6 |
| | 16 | ― | ― | ― | 0.065 | ― | ― | 58.8±2.5 | 99.5±4.5 |
| 17 | | 0.010 | 0.034 | 0.003 | 0.065 | ― | 0.084 | 13.2±0.8 | 22.3±1.3 |
| | 17 | ― | ― | ― | ― | ― | ― | 59.1±3.2 | 100.0 |
| 18 | | 0.010 | 0.034 | 0.003 | 0.065 | 0.003 | 0.087 | 11.9±2.1 | 20.2±3.7 |
| | 18 | ― | ― | ― | ― | 0.003 | ― | 58.2±1.4 | 98.5 ±2.5 |
| 19 | | 0.010 | 0.034 | 0.003 | 0.065 | 0.005 | 0.089 | 6.8±2.0 | 11.6±3.5 |
| | 19 | ― | ― | ― | ― | 0.005 | ― | 56.7±2.1 | 96.0±3.7 |
| 20 | | 0.010 | 0.034 | 0.003 | 0.065 | 0.007 | 0.091 | 4.5±0.3 | 7.7±0.6 |
| | 20 | ― | ― | ― | ― | 0.007 | ― | 56.3 ±2.6 | 95.2 ±4.8 |

The synergistic effect of compositions of Examples 1-20 comprising five active ingredients is shown in **Figure 1,** where amounts of Sp2/0-Ag14 cells expressed in percentage of amounts of untreated cells (the amount of untreated cells is 100 %) are shown on the vertical axis. On the horizontal axis amounts expressed in % by mass of L-tryptophan, 2-deoxy-D-ribose, adenine, malic acid and L-ascorbic acid are shown. White columns show the amounts of cells expressed in percentage of the amounts of untreated cells after 48 hr incubation using the above-mentioned substances alone and black columns show the results obtained when they were used together with the other four active components. The changes in different degrees (in cases of L-tryptophan even in opposite direction) of the white and black columns with increasing amounts of the individual substances prove the synergistic effect, and the black columns demonstrate the powerful tumour-cell-killing effect of the compositions.

On the basis of Examples 21-33 Table 2 shows how the effect of a composition comprising five active components will increase in case of Sp2/0-Ag14 cells when further components are added. In Table 2 the amounts of materials expressed in % by mass and multiplied by 1000 are shown, thus the numbers in the Table should read by multiplying with 10⁻³; for example in Example 21 in case of L-tryptophan the number 6 means 6 x 10⁻³, that is 0.006 % by mass. The conditions of the experiments were the same as before.

Similarly to Table 1 also here it can be seen from data of the two last columns of Table 2 (in number of cells and in %) that using the above-mentioned substances alone no one has cell-proliferation-decreasing effect, moreover by the effect of L-phenylalanine (Control 22), L-arginine (Control 23), L-histidine (Control 24) and riboflavin (Control 29) the number of cells as compared to the number of untreated cells (Control 21) even slightly increased. It can also be seen that the same substances when used in the same amounts synergistically increase the effect of composition of Example 21. Without potentiating substances the effects of Example 21 comprising five active ingredients were in all cases significantly less than together with the other compounds.

The effects of compositions of Examples 103-107 comprising seventeen active ingredients and the effects of compositions of Examples 98-102 comprising five active ingredients having the same amounts of the common active ingredients have also been compared. To help the comparability with the earlier results Sp2/0-Ag14 cells were also used and the experimental conditions were the same as before. The results are shown in **Figure 2**. On the vertical axis the changes of the amounts of cells expressed in the percentage of the amounts of untreated cells are shown. On the horizontal axis the changes expressed in % of the compositions (changes of the amounts of the active ingredients) are shown. The composition of Example 98 comprising five active ingredients is regarded as 100 %. Thus, the 80 % composition of Example 99 contains every component in an amount of 80 %. In cases of compositions comprising seventeen active ingredients the composition of Example 103 is regarded as 100 %.

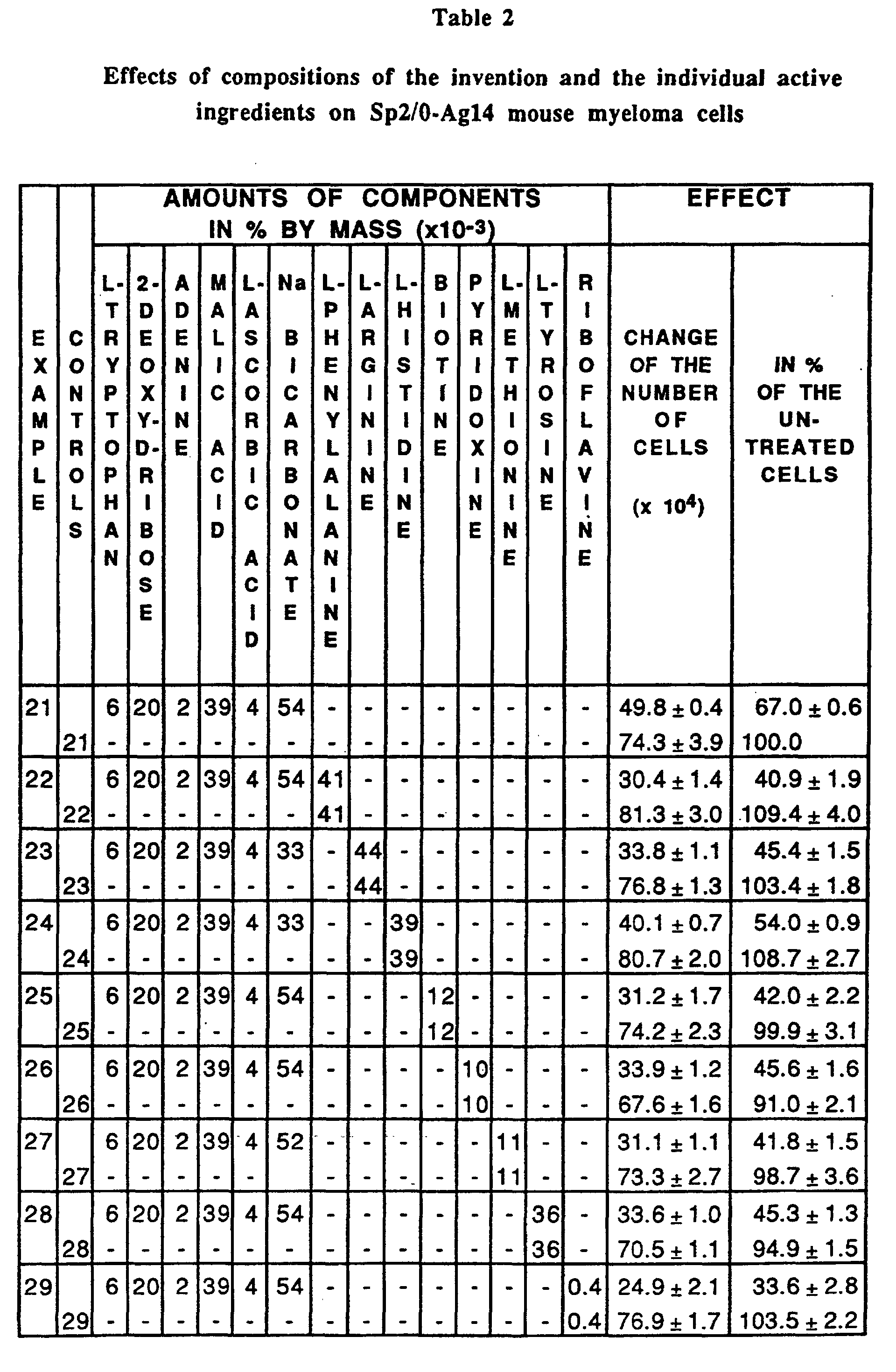

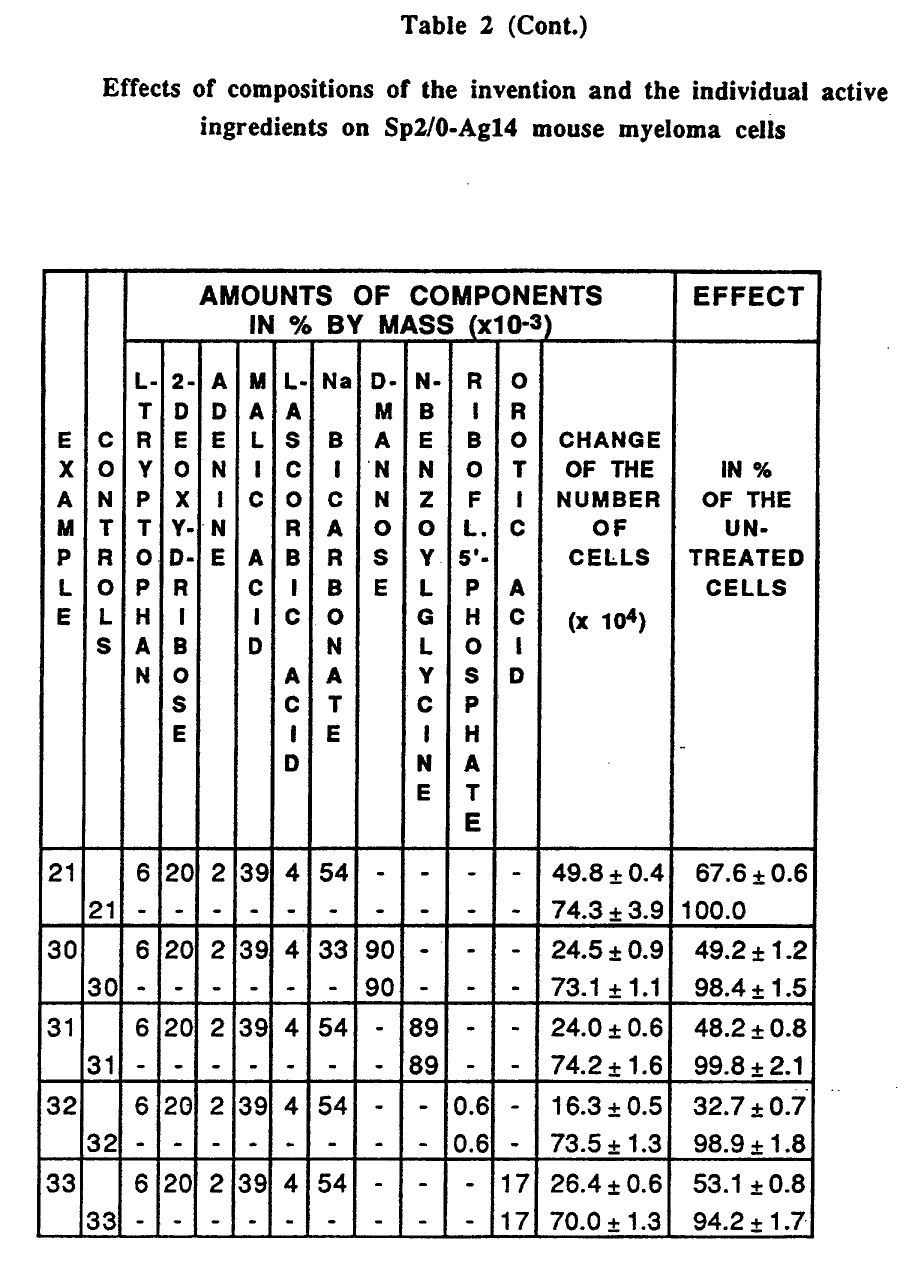

To exclude the possibility that the measured effect is a result of an osmotic effect or an aspecific overload of nutrients or an amino acid imbalance, control mixtures were also prepared. These control mixtures contained substances which in previous experiments proved to be ineffective in PADS. The composition of the control mixtures was given in connection with Table 1. The results obtained from experiments with the compositions of Examples 98-102 and 103-107 and with the control mixtures are shown in Figure 2 where the line with signs of open circles shows the change of cell amounts after 48 hr incubation in case of control mixtures, the line with signs of filled circles shows the change in case of compositions comprising five active ingredients of Examples 98-102 and the line with signs of open triangles shows the change in case of compositions comprising seventeen active ingredients of Examples 103-107. In the experiments carried out with the 100 % compositions of Examples 98 and 103 and shown in Figure 2 the individual active ingredients were used in such amounts which do not influence substantially the cell number (as it can be seen from the results of the earlier experiments shown in Tables 1 and 2). It can be seen from Figure 2 that these components when used together have a significant antitumour effect. This fact once again proves the synergistic manner of the co-operation of the active ingredients. It can also be seen from Figure 2 that the control mixtures do not exert any substantial effect on the number of cells. Thus, based on these experiments it can be excluded that the measured effects are the result of an osmotic effect or an aspecific overload of nutrients or an amino acid imbalance.

To find out whether counter-ions have any role in the effects of the compositions experiments were carried out where K⁺ (Examples 34-63) or Ca⁺⁺ (Examples 64-93) ions were used instead of Na⁺ ions. There were no significant differences between the effects of compositions using different counter-ions, in fact the results were exactly the same.

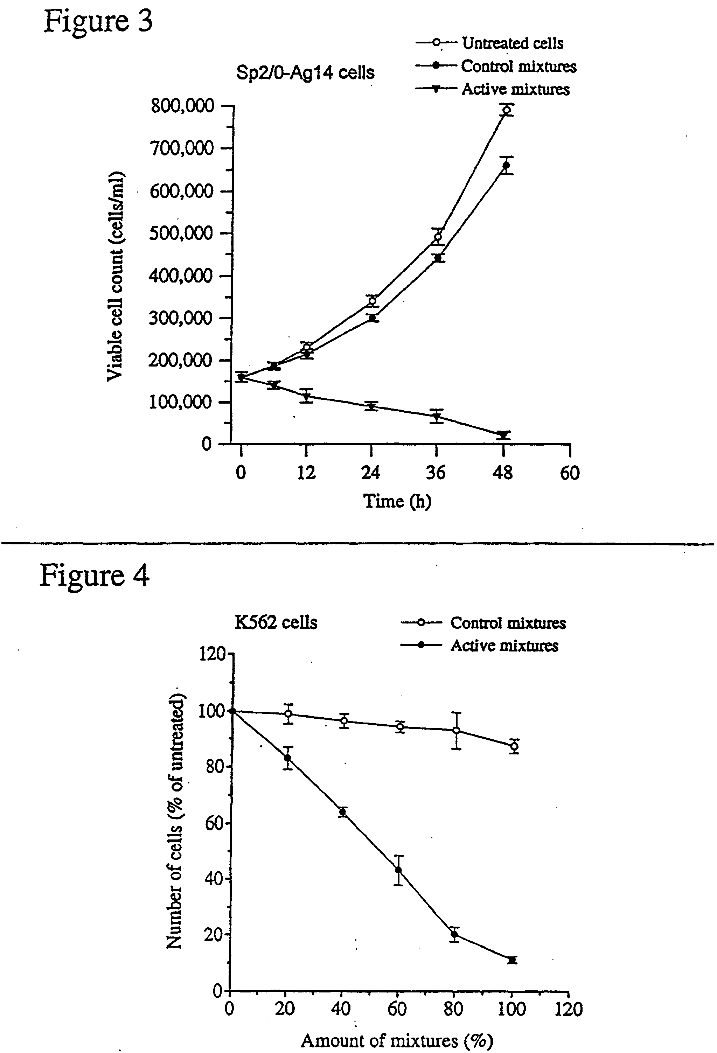

As a function of time the effect of the 100 % composition of Example 103 on Sp2/0-Ag14 cells was investigated and compared to the 100 % control mixture and to untreated cells. The experimental conditions were the same as in the previous experiments except that in this case the cells were counted not only after 48 hr but also after 6; 12; 24; and 36 hr. The results of these kinetic experiments are shown in **Figure 3.** On the vertical axis the cell number/ml is shown. On the horizontal axis the time is shown in hours. The line with signs of open circles shows the change of the number of cells in case of untreated cells, the line with signs of filled circles shows the change in case of the control mixture and the line with signs of filled triangles shows the change in case of compositions of Example 103 comprising seventeen active ingredients as a function of time. It can be seen from Figure 3 that while the control mixture does not have any substantial effect on the cell number since it increases exponentially with time and the shape of the line corresponds to that of the line showing the proliferation of untreated cells, the composition according to the invention is toxic for the tumour cells causing a decrease in their number with time. Thus, the composition used according to the invention does not only inhibit cell proliferation but it has a cytotoxic effect. The amounts of the active ingredients in the above-mentioned composition comprising seventeen substances were chosen so as not to have any significant effect on tumour cells when taken separately. (Naturally, this is even more true for the 80 %, 60 % etc. compositions where the amounts of the active agents are by 20 %, 40 % etc. less.) Since the same compositions were used in all *in vitro* experiments, the results prove in all cases the synergistic co-operation of the active components.

The above results prove that the effect is caused by the synergistic action of substances selected according to the recognitions constituting the basis of the invention. Furthermore, these results also prove that the recognitions are correct. Since the various cancer cells differ from normal cells in different degrees, the most effective compositions used according to the invention against cancer cells differ from each other as well. This means that against every tumour the most adequate composition can be selected using the synergistic tumour-cell-killing effect as criteria. However for prevention or when the type of the tumour can not be verified or time is running out, it is preferable to use the general compositions comprising seventeen active ingredients. Thus, in the next experiments it was investigated how general the effect of these compositions is and how their efficiency is.

The effect of the compositions of Example 103-107 comprising seventeen active ingredients on K-562 human erythroleukaemia cells was investigated and compared to the effect of the control mixture mentioned above. The results of the experiments are shown in **Figure 4.** Similarly to Figure 2, on the vertical axis the changes of the cell amounts expressed in percentage of the amounts of untreated cells is shown. On the horizontal axis the changes expressed in % of the compositions (changes of the amounts of the active ingredients) are shown. The line with signs of open circles shows the effects of the control mixtures, while the line with signs of filled circles shows the effects of compositions comprising seventeen active ingredients. It can be seen from Figure 4 that the compositions used according to the invention have a significant tumour-cell-killing effect in case of K-562 human erythroleukaemia cells, too.

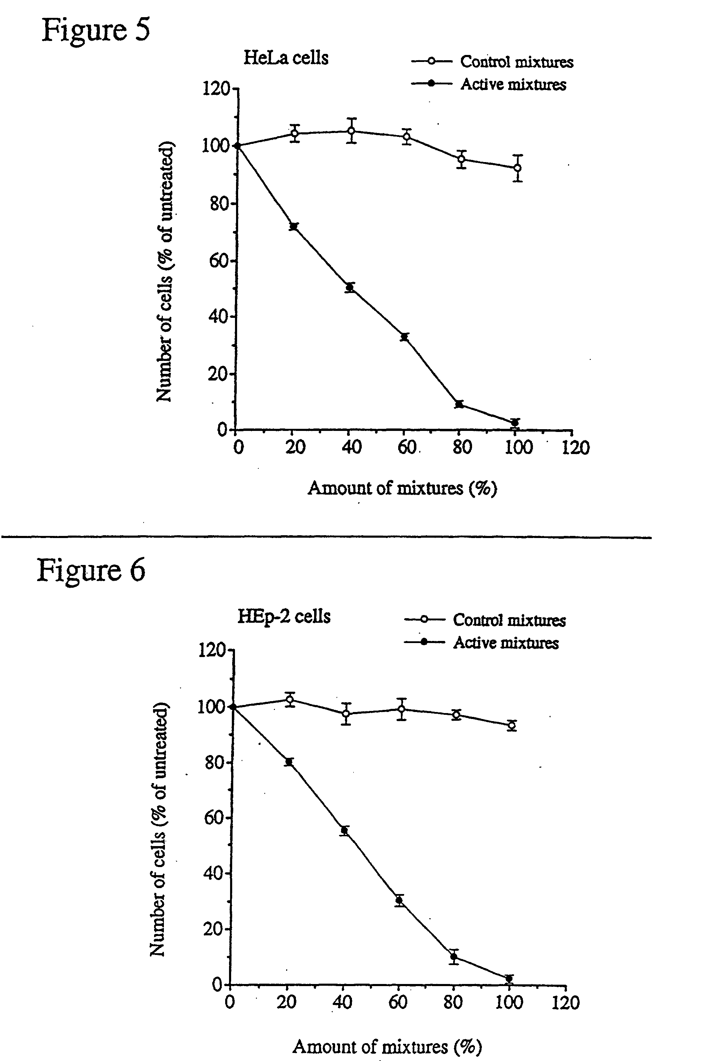

Thereafter the effect of the compositions of Examples 103-107 comprising seventeen active ingredients was investigated in case of HeLa human epitheloid carcinoma cervix cells and HEp-2 human epidermoid carcinoma larynx cells and compared to the effect of the control mixture. This experiment was necessary to show whether the previous results were general and independent of the perturbing effects of the medium used, of the suspension method, of the counting and of the detection. The survival of HeLa, HEp-2 and Vero cells was measured by assessing the endogenous alkaline phosphatase activity of cells. Briefly, after incubation period, non-adherent (presumably damaged) cells were eliminated by washing. Then 150 µg of alkaline phosphatase substrate (4-nitrophenylphosphate, Sigma tablets No. 111-112) dissolved in 150 µl of fresh 10 % diethanolamine buffer (pH 9.8) were added to each well. Plates were incubated at 30 °C until the absorbance in the case of untreated cells reached a value of about 1. The reaction was stopped by adding 50 µl of 3 M NaOH to each well. The absorbance was measured at 405 nm with the aid of a Dynatech ELISA reader. Backround values were subtracted from each reading. The results are shown in **Figure 5** (in case of HeLa cells) **and Figure 6** (in case of HEp-2 cells). Similarly to Figures 2 and 4, in both Figures the vertical axis shows the change of the amounts of cells expressed in the percentage of, the amounts of untreated cells. The change of the cell number in these cases was detected by measuring the extinction. The horizontal axis shows the changes expressed in % of the compositions (changes in the amounts of the active ingredients). In both Figures 5 and 6 the lines with signs of open circles show the effects of the control mixtures, while the lines with signs of filled circles show the effects of compositions of Examples 103-107 comprising seventeen active ingredients. It can be seen from the Figures that the compositions are effective against both cell lines. This result is even more valuable considering that HEp-2 is known as a "hardy cell line" resistant to environmental effects [(*American Type Culture Collection Catalogue of Cell Lines and Hybridomas, 5th ed., pp. 15-16 (1985)*].

According to the theoretical bases of the invention, the compositions selectively effect only cancer cells. To further prove this statement, the effect of compositions of Example 103-107 comprising seventeen active ingredients and of control mixtures on normal cell line Vero African green monkey kidney cells was investigated, too. The results of the experiment are shown in **Figure 7.** On the vertical axis the change of the amount of cells expressed in percentage of cell amount of untreated cells is shown. On the horizontal axis the changes expressed in % of the compositions (changes of the amounts of the active ingredients) are shown. The line with signs of open circles shows the effects of control mixtures while the line with signs of filled circles shows the effects of compositions of Example 103-107 comprising seventeen active ingredients. It can be seen from Figure 7 that the compositions used according to the invention do not exert cytotoxic effects on normal Vero cell line, because the effect of the 100 % composition of Example 103 on the proliferation would be, when plotted against time, similar to that shown in Figure 3 in case of control mixtures. Vero being a fast proliferating cell line [*American Type Culture Collection Catalogue of Cell Lines and Hybridomas*, *5th ed*., *pp*. *45-46 (1985)],* this experiment proves that, contrary to the cytostatics, the composition used according to the invention is not toxic for all the fast proliferating cells, but it influences selectively only tumour cells. Based on the above-mentioned results the compositions used according to the invention can be regarded as antineoplastic, and not as cytotoxic, preparations.

For proving the *in* vivo effectiveness of the composition used according to the invention pharmacological experiments were carried out as well. During these experiments methods and conditions approved by the scientific literature were always used. The experiments were carried out with Sp2/0-Ag14 cells. For the experiments 5-6 weeks old female, BALB/c mice were used. The experiments were carried out with two groups comprising 10 mice each. Into the mice 5·10⁴ Sp2/0-Ag14 cells suspended in 200 µl of incomplete RPMI 1640 medium were injected i.p. The day of injection was regarded as the 0 day of the experiment. The cell number necessary for the experiments was determined by tumorigenicity test. The treatment of the animals began 24 hr after the injection of the cells (this was the first day of the experiment) and continued for 10 consecutive days. The control group was injected i.p. with 200 µl of PBS (phosphate buffered saline) at every treatment, while the treated group was injected i.p. with the same volume of the composition of Example 108 comprising seventeen active ingredients.

The most effective way of treatment would have been a continuous infusion keeping the concentration of the components of the composition on a fairly high leveL Because of practical difficulties, an approximately continuous, periodical treatment was used i.e. the animals were treated six times per day. The results of the experiments are shown in **Figure 8.** On the horizontal axis the number of days passed from the beginning of the experiment is shown. On the vertical axis the values of the survival %, i.e. the number of mice being alive on the given day, are shown. Thus 10 mice means 100 % and 8 mice means 80 %, respectively. White columns show the survival of treated mice, black columns that of untreated mice. It can be seen from Figure 8 that e. g. on the 16th day no one untreated mouse was alive and all treated mice were still alive. All the untreated mice died by the 16th day, the treated ones only on the 25th day. It can be seen that due to the treatment a significant increase in the lifespan can be achieved. The median survival time was 21 days for the treated group while 14 days for the untreated group. The T/C % value calculated from these median survival times (150 %) is much higher than 125% approved as criterion for effectiveness. According to the literature, based upon this value the composition used according to the invention can be regarded as.a preparation with powerful antitumour effect against the given cell line.

In order to prove that the increase of survival time is a result of the toxic effect of the composition of Example 108 on tumour cells and not of a strong roborating effect, another experiment was carried out. 10-10 mice were treated in the same way. After a 10-day treating period the cells were removed from the abdominal cavities of both groups of mice and then counted. The difference between the average cell numbers (5.55 x 10⁵ and 5.95 x 10⁷, resp.) is significant (p<0.001) and proves the in vivo effectiveness of the composition according to the invention.

To prove further the effectiveness of the composition used according to the invention and to exclude the possibility that the results are only the consequences of a local effect (the cells were in the abdominal cavity and treated i.p.), another experiment was conducted with congenitally immune deficient (thymus deficient) (nude) mice injected s.c. with human solid tumour. For the experiment HeLa cells and 6-8-week old female BALB/c (nu/nu) mice were used and 5 x 10⁶ cells were injected s.c. in the lower extremities of the mice. The treatment started with the composition of Example 108 when the size of the tumours reached the average volume of 50 mm³. The treatment schedule, the time of the treatment and the solutions (Example 108) used for the treatment were the same as in the previous pharmacological experiment. Sizes of the tumours [(length (L), width (W), height (H)] were measured twice a week with digital callipers (Mitutoyo, Inc., Tokyo, Japan). Volumes of the tumours (calculated by the equation LWH/2) and also the relative tumour volumes Vₜ/V₀ [*Eur. J. Cancer. Clin. Oncol., 21, 1253-1260 (1988)]* where Vₜ is the actual tumour volume and V₀ is the tumour volume at the beginning of the treatment were calculated. The results are summarized in **Figure 9**. On the vertical axis the average of the Vₜ/V₀ values while on the horizontal axis the days after starting the treatment are shown. The line with signs of open circles shows the relative tumour volume increase after the beginning of the treatment in case of control mice and the line with signs of filled circles shows the same in case of treated animals. It can be seen from Figure 9 that the treatment considerably slowed down the tumour growth. For example in case of control mice the tumour volume increased during 16.5 days to ninefold value whereas in case of treated mice for the same ninefold growth 33 days were needed. The T/C % was also calculated from Vₜ/V₀ values of treated and control groups. The values of T/C % were in all cases below 42% which is acceptable as criteria of effectiveness [*Europ. J*. *Cancer, 17, 129-142 (1981)*]. The experiment shows once again that the compositions according to the invention have significant antitumour effects. The results are particularly promising considering that due to technical problems we were far from the optimum treating procedure and the maximum tolerable dose and that the same treatments in clinical conditions could be performed more effectively (longer treating time, permanent effect e.g. by infusion etc.).

Body-weight change during a treatment is a good and generally used index to characterize the toxicity of the treatment; therefore, the weight of the animals was measured during the above experiments. According to the literature, the average body weight decreases by 10-15 %, the preparation is regarded to be toxic [*Eur. J. Cancer. Clin. Oncol., 21, 1253-1260 (1988)*]. In our experiment, the change in average body weight was -5.9±4.1 % in case of the control group and -6.6±3.9 % in case of the treated group. Thus, there is no significant difference between the two groups and this means that the composition according to the invention has no toxic side-effect.

To prove the preventive usefulness of the composition used according to the invention 2-2 groups of BALB/c mice (10 mice per group) were treated in such a way that the food of the treated animals was mixed in 1:1 ratio with the compositions of Examples 109-117 and the animals were fed ad libitum. 5 days after starting the treatment 1 x 10⁴ Sp2/0-Ag14 cells were inoculated i.p. into both groups of animals. The treatment was continued for 100 days. Mice fed with the 1-1 mixture of compositions of Examples 109-117 and with normal food showed no evidence of i.p. tumour growth when sacrified 100 days following injection of the cells. At the same time the survival times of the control animals in the different experimental groups ranged from of 21 to 26 days following injection of Sp2/0-Ag14 cells.

Depending on the dose and the way of treatment the compositions used according to the invention can be utilized for prevention of cancerous diseases, for inhibiting tumour development in case of AIDS and organ transplantations, for hindering the formation of metastases and for adjuvant, combined and direct therapy of tumorous patients.

The main advantages of the compositions used according to the invention are as follows:
a) Using an adequate dosage the compositions can be applied for prevention of cancerous diseases, for inhibition of tumour formation in case of AIDS and transplantations, for hindering metastasis formation and for direct, adjuvant or combined treatment and cure of cancerous diseases.
b) They are not toxic. Toxicity experiments, based on measuring the loss of body weight of animals proved that the compositions are not toxic. According to literature data, toxicity of the individual components of the compositions is very low, and even this low toxicity decreases when these substances are used together. Besides, primates tolerate these substances better than small animals used for the determination of toxicity values. Considering all these facts it can be stated that the compositions should be regarded as not toxic.
c) They have no or only negligible adverse effects. A lot of observations have been gathered about the side-effects of the components because almost all of them (except 2-deoxy-D-ribose) have been used in some kind of therapy (in anticancer therapy only ascorbic acid) or as a food supplement. According to experiences gathered in these applications it is fully clear that the compositions practically have no side-effects.
d) They are selective. The selectivity is proved by own experiments showing that compositions toxic for various cancer cells are not toxic for normal Vero cells and for the experimental animals or for their normal cells, respectively. This selectivity is enhanced by the fact that there are much more normal cells than cancer cells and the latter cells accumulate the components of the compositions while in case of normal cells their uptake is regulated.
e) They can be widely used, their effects are general. The effects of the compositions were investigated in case of mouse myeloma (Sp2/0-Ag14), human erythroleukaemia (K-562), human epitheloid carcinoma cervix (HeLa) and human epidermoid carcinoma larynx (HEp-2) cell lines. These cells represent a broad spectrum because they involve both human and animal tumours and even from human tumours a leukaemia and two largely different solid tumours are represented. The results obtained from experiments with these cell lines support the above statement that the compositions have general effects and can be widely used.
f) They can be prepared without any difficulty. The components of the compositions are substances with low molecular weight and are readily available, so the compositions are easy to prepare.
g) They are soluble in water and thus easy to dose.

## Claims

1. Use of a composition for preparing medicaments for preventing and treating cancerous diseases, the said composition comprising at least three active compounds occurring in the circulatory system: at least one amino acid, at least one vitamin and at least one member selected from the group consisting of adenine, 2-deoxy-D-ribose, D-mannose, malic acid and/or their pharmaceutically acceptable salts, with the proviso that if the composition contains beside amino acid(s) only malic acid and a vitamin, the vitamin may be only other than ascorbic acid.

2. The use as claimed in claim 1, wherein beside the active compounds also carriers, diluents and/or other auxiliary agents commonly employed in pharmacy are employed.

3. The use as claimed in claim 1, wherein L-methionine, L-tryptophan, L-tyrosine, L-phenylalanine, L-arginine, L-histidine, N-benzoylglycine and/or a salt thereof is employed as amino acid.

4. The use as claimed in claim 1, wherein d-biotin, pyridoxine, riboflavin, riboflavin-5'-phosphate, L-ascorbic acid, orotic acid and/or a salt thereof is employed as vitamin.

5. The use as claimed in claim 1, wherein 0.002-70% by mass of at least one member selected from the group consisting of L-methionine, L-tryptophan, L-tyrosine, L-phenyl-alanine, L-arginine, L-histidine and/or their salts is employed as amino acid, 0.0004-80% by mass of at least one member selected from the group consisting of d-biotin, pyridoxine, riboflavin, riboflavin-5'-phosphate, L-ascorbic acid and/or their salt is employed as vitamin and 0.003-80% by mass of at least one member selected from the group consisting of adenine, orotic acid, N-benzoylglycine, 2-deoxy-D-ribose, D-mannose, malic acid and/or their pharmaceutically acceptable salts is employed.

6. The use as claimed in claim 1, wherein 0.9-25% by mass of L-methionine, 0.8-19% by mass of L-tryptophan, 1.1-48% by mass of L-arginine, 0.9-46% by mass of d-biotin, 1.2-16% by mass of pyridoxine, 0.03-42% by mass of riboflavin-5'-phosphate, 0.05-18% by mass of D-glucosamine, 0.5-60% by mass of 2-deoxy-D-ribose, 0.7-68% by mass of malic acid, 0.6-40% by mass of D-mannose and/or their pharmaceutically acceptable salts are employed.

7. The use as claimed in claim 1, wherein 0.005-34% by mass of L-methionine, 0.002-25% by mass of L-tryptophan, 0.02-23% by mass of L-tyrosine, 0.04-30% by mass of L-phenylalanine, 0.04-50% by mass L-arginine, 0.03-34% by mass of L-histidine, 0.05-22% by mass of N-benzoylglycine, 0.01-60% by mass of d-biotin, 0.01-20% by mass of pyridoxine, 0.0004-45% by mass of riboflavin, 0.0005-45% by mass of riboflavin-5'-phosphate, 0.003-70% by mass of L-ascorbic acid, 0.004-15% by mass of lipoic acid, 0.01-17% by mass of orotic acid, 0.001-10% by mass of adenine, 0.01-63% by mass of 2-deoxy-D-ribose, 0.08-42% by mass of D-mannose, 0.05-20% by mass of D-glucosamine, 0.01-80% by mass of malic acid, 0.02-60% by mass of oxalacetic acid, 0.001-10% by mass of adenosine triphosphate and/or their pharmaceutically acceptable salts are employed.

8. The use as claimed in claim 1, wherein 30-44% by mass of L-arginine, 27-35% by mass of riboflavin-5'-phosphate, 38-62% by mass of malic acid and/or their pharmaceutically acceptable salts are employed.

9. The use as claimed in claim 1 for preparing medicaments in the form of tablets or capsules, for oral administration.

10. The use as claimed in claim 1 for preparing medicaments in the form of an infusion solution for parenteral administration.

11. The use as claimed in claim 10 for preparing 0.5 to 3 l of an infusion solution comprising 10 to 200 g/l of the active composition.

12. The use as claimed in claim 10 for preparing an infusion solution comprising 50 to 190 g/l of the active composition.

13. The use as claimed in claim 10 for preparing an infusion solution comprising 150 to 180 g/l of the active composition.

14. Pharmaceutical compositions comprising 30-44% by mass of L-arginine, 27-35% by mass of riboflavin-5'-phosphate, 38-62% by mass of malic acid and/or their pharmaceutically acceptable salts.

## Patentansprüche

1. Verwendung einer Komposition zum Vorbeugen und zur Behandlung von Krebserkrankungen, welche Komposition von den im Kreislauf vorkommenden aktiven Verbindungen wenigstens drei enthält: wenigstens eine Aminosäure, wenigstens ein Vitamin und wenigstens eine von den folgenden Verbindungen: Adenin, 2-Desoxy-D-ribose, D-Mannose, Apfelsäure und/oder die physiologisch verträglichen Salze der aufgeführten Stoffe, mit der Einschränkung, dass falls die Komposition ausser einer Aminosäure nur Apfelsäure und ein Vitamin enthält, dieses Vitamin ein anderes als L-Ascorbinsäure ist.

2. Verwendung nach Anspruch 1, worin neben den Wirkstoffen in der Arzneimittelherstellung übliche Trägerstoffe, Verdünnungsmittel und/oder sonstige Hilfsstoffe verwendet werden.

3. Verwendung nach Anspruch 1, worin als Aminosäure L-Methionin, L-Tryptophan, L-Tyrosin, L-Phenylalanin, L-Arginin, L-Histidin, N-Benzoylglycin und/oder deren Salze verwendet werden.

4. Verwendung nach Anspruch 1, worin als Vitamin d-Biotin, Pyridoxin, Riboflavin, Riboflavin-5'-phosphat, L-Ascorbinsäure, Orotsäure und/oder deren Salze verwendet werden.

5. Verwendung nach Anspruch 1, worin in einer Menge von 0,002-70 Masse% wenigstens eine Verbindung aus der Gruppe L-Methionin, L-Tryptophan, L-Tyrosin, L-Phenylalanin, L-Arginin, L-Histidin und/oder deren Salze als Aminosäure, in einer Menge von 0,0004-80 Masse% wenigstens eine Verbindung aus der Gruppe d-Biotin, Pyridoxin, Riboflavin, Riboflavin-5'-phosphat, L-Ascorbinsäure und/oder deren Salze als Vitamin und in einer Menge von 0,003-80 Masse% wenigstens eine Verbindung aus der Gruppe Adenin, Orotsäure, N-Benzoylglycin, 2-Desoxy-D-ribose, D-Mannose, Apfelsäure und/oder deren physiologische verträglichen Salze verwendet werden.

6. Verwendung nach Anspruch 1, worin 0,9-25 Masse% L-Methionin, 0,8-19 Masse% L-Tryptophan, 1,1-48 Masse% L-Arginin, 0,9-46 Masse% d-Biotin, 1,2-16 Masse% Pyridoxin, 0,03-42 Masse% Riboflavin-5'-phosphat, 0,05-18 Masse% D-Glucoseamin, 0,5-60 Masse% 2-Desoxy-D-ribose, 0,7-68 Masse% Apfelsäure, 0,6-40 Masse% D-Mannose und/oder die pharmazeutisch annehmbaren Salze dieser Verbindungen verwendet werden.

7. Verwendung nach Anspruch 1, worin 0,005-34 Masse% L-Methionin, 0,002-25 Masse% L-Tryptophan, 0,02-23 Masse% L-Tyrosin, 0,04-30 Masse% L-Phenylalanin, 0,04-50 Masse% L-Arginin, 0,03-34 Masse% L-Histidin, 0,05-22 Masse% N-Benzoylglycin, 0,01-60 Masse% d-Biotin, 0,01-20 Masse% Pyridoxin, 0,0004-45 Masse% Riboflavin, 0,0005-45 Masse% Riboflavin-5'-phosphat, 0,003-70 Masse% L-Ascorbinsäure, 0,004-15 Masse% Lipoinsäure, 0,01-17 Masse% Orotsäure, 0,001-10 Masse% Adenin, 0,01-63 Masse% 2-Desoxy-D-ribose, 0,08-42 Masse% D-Mannose, 0,05-20 Masse% D-Glucoseamin, 0,01-80 Masse% Apfelsäure, 0,02-60 Masse% Oxalessigsäure, 0,001-10 Masse% Adenosintriphosphat und/oder die physiologisch verträglichen Salze dieser Verbindungen verwendet werden.

8. Verwendung nach Anspruch 1, worin 30-44 Masse% L-Arginin, 27-35 Masse% Riboflavin-5'-phosphat, 38-62 Masse% Apfelsäure und/oder ihre pharmazeutisch annehmbaren Salze verwendet werden.

9. Verwendung nach Anspruch 1 zur Herstellung von Heilmitteln in Form von Tabletten oder Kapseln für orale Verabreichung.

10. Verwendung nach Anspruch 1 zur Herstellung von Heilmitteln in Form von einer Infusionslösung für parenterale Verabreichung.

11. Verwendung nach Anspruch 10 zur Herstellung von 0,5 bis 3 L einer Infusionslösung, die 10 bis 200 g/l aktive Komposition enthält.

12. Verwendung nach Anspruch 10 zur Herstellung einer Infusionslösung, die 50 bis 190 g/l aktive Komposition enthält.

13. Verwendung nach Anspruch 10 zur Herstellung einer Infusionslösung, die 150 bis 180 g/l aktive Komposition enthält.

14. Pharmazeutische Kompositionen enthaltend 30-44 Masse% L-Arginin, 27-35 Masse% Riboflavin-5'-phosphat, 38-62 Masse% Apfelsäure und/oder ihre pharmazeutisch annehmbaren Salze.

## Revendications

1. Mise en oeuvre d'une composition propre à prévenir et à traiter des maladies cancéreuses et qui contient au moins trois des composés actifs présents dans la circulation sanguine: au moins un acide aminé, au moins une vitamine et au moins un des composés suivants: de l'adénine, de la 2-désoxy-D-ribose, de la D-mannose, de l'acide malique et/ou les sels convenables du point de vue physiologique des substances énumérées, avec cette restriction que si la composition ne contient, en plus d'un acide aminé, que de l'acide malique et une vitamine, cette vitamine sera autre que de l'acide L-ascorbique.

2. Mise en oevre selon la Revendication 1, **caractérisée en ce que** l'on met en oeuvre en plus des substances actives, des supports généralement utilisés dans la préparation de produits pharmaceutiques, des agents dissolvants et/ou d'autres substances auxiliaires.

3. Mise en oeuvre selon la Revendication 1, **caractérisée en ce que** l'on met en oeuvre à titre d'acide aminé de la L-méthionine, du L-tryptophane, de la L-tyrosine, de la L-phénylalanine, de la L-arginine, de la L-histidine, de la N-benzoylglycine et/ou les sels de ceux-ci.

4. Mise en oeuvre selon la Revendication 1, **caractérisée en ce que** l'on met en oeuvre à titre de vitamine de la d-biotine, de la pyridoxine, de la riboflavine, du 5'-phosphate de riboflavine, de l'acide L-ascorbique, de l'acide orotique et/ou les sels de ceux-ci.

5. Mise en oeuvre selon la Revendication 1, **caractérisée en ce que** l'on met en oeuvre à titre d'acide aminé une quantité de 0,002-70 % en masse d'au moins un composé du groupe L-méthionine, L-tryptophane, L-tyrosine, L-phénylalanine, L-arginine, L-histidine et/ou les sels de ceux-cià, titre d'acide aminé, une quantité de 0,0004-80 % en masse d'au moins un composé du groupe d-biotine, pyridoxine, riboflavine, 5'-phosphate de riboflavine, acide L-ascorbique et/ou les sels de ceux-ci à titre de vitamine, et une quantité de 0,003-80 % en masse d'au moins un composé du groupe adénine, acide orotique, N-benzoylglycine, 2-désoxy-D-ribose, D-mannose, acide malique et/ou leurs sels convenables du point de vue physiologique.

6. Mise en oeuvre selon la Revendication 1, **caractérisée en ce que** l'on met en oeuvre 0,9-25 % en masse de L-méthionine, 0,8-19 % en masse de L-tryptophane, 1,1-48 % en masse de L-arginine, 0,9-46 % en masse de d-biotine, 1,2-16 % en masse de pyridoxine, 0,03-42 % en masse de 5'-phosphate de riboflavine, 0,05-18 % en masse de l'amine de D-glucose, 0,5-60 % en masse de 2-désoxy-D-ribose, 0,7-68 % en masse d'acide malique, 0,6-40 % en masse de D-mannose et/ou leurs sels acceptables du point de vue pharmaceutiques.

7. Mise en oeuvre selon la Revendication 1, **caractérisée en ce que** l'on met en oeuvre 0,005-34 % en masse de L-méthionine, 0,002-25 % en masse de L-tryptophane, 0,02-23 % en masse de L-tyrosine, 0,04-30 % en masse de L-phénylalanine, 0,04-50 % en masse de L-arginine, 0,03-34 % en masse de L-histidine, 0,05-22 % en masse de N-benzoylglycine, 0,01-60 % en masse de d-biotine, 0,01-20 % en masse de pyridoxine, 0,0004-45 % en masse de riboflavine, 0,0005-45 % en masse de 5'-phosphate de riboflavine, 0,003-70 % en masse d'acide L-ascorbique, 0,004-15 % en masse d'acide lipoïque, 0,01-17 % en masse d'acide orotique, 0,001-10 % en masse d'adénine, 0,01-63 % en masse de 2-désoxy-D-ribose, 0,08-42 % en masse de D-mannose, 0,05-20 % en masse d'amine de D-glucose, 0,01-80 % en masse d'acide malique, 0,02-60 % en masse d'acide oxalacétique, 0,001-10 % en masse de triphosphate d'adénosine et/ou les sels convenabales du point de vue physiologique de ces composés.

8. Mise en oeuvre selon la Revendication 1, **caractérisée en ce que** l'on met en oeuvre 30-44 % en masse de L-arginine, 27-35 % en masse de 5'-phosphate de riboflavine, 38-62 % en masse d'acide malique et/ou leurs sels acceptables du point de vue pharmaceutique.

9. Mise en oeuvre selon la Revendication 1 afin de préparer des substances médicamenteuses sous forme de comprimés ou de capsules pour administration par voie orale.

10. Mise en oeuvre selon la Revendication 1 afin de préparer des substances médicamenteuses sous forme d'une solution pour infusion pour administration par voie parentérale.

11. Mise en oeuvre selon la Revendivation 10 afin de préparer 0,5 à 3 litres d'une solution pour infusion qui contient 10 à 200 g/l de la composition active.

12. Mise en oeuvre selon la Revendication 10 afin de préparer une solution pour infusion qui contient 50 à 190 g/l de la composition active.

13. Mise en oeuvre selon la Revendication 10 afin de préparer une solution pour infusion qui contient 150 à 180 g/l de la composition active.

14. Compositions pharmaceutiques contenant 30-44 % en masse de L-arginine, 27-35 % en masse de 5'-phosphate de riboflavine, 38-62 % en masse d'acide malique et/ou leurs sels acceptables du point de vue pharmaceutique.
